# EUROPÄISCHE PATENTSCHRIFT

(11) **EP 1 558 204 B1**
(45) Veröffentlichungstag und Bekanntmachung des Hinweises auf die Patenterteilung: **12.10.2011**
(21) Anmeldenummer: 03758007.3
(22) Anmeldetag: 17.10.2003
(51) Int. Cl.: A61K 8/41, A61Q 5/10

(54) **2-AMINO-5-ETHYLPHENOL ENTHALTENDES MITTEL ZUM FÄRBEN VON KERATINFASERN**
MEANS FOR COLOURING KERATIN FIBRES COMPRISING 2-AMINO-5-ETHYLPHENOL
AGENTS CONTENANT DU 2-AMINO-5-ETHYLPHENOL POUR LA COLORATION DE FIBRES KERATINIQUES

(30) Priorität: 07.11.2002 DE 10251830
(43) Veröffentlichungstag der Anmeldung: 03.08.2005
(73) Patentinhaber: Wella GmbH, 65825 Schwalbach am Taunus (DE)
(72) Erfinder: PASQUIER, Cécile, CH-1723 Marly (CH); BUCLIN, Véronique, CH-1638 Morlon (CH); BRAUN, Hans-Jürgen, CH-3182 Ueberstorf (CH); SAUTER, Guido, CH-3303 Jegenstorf (CH)
(86) Internationale Anmeldenummer: PCT/EP2003/011526
(87) Internationale Veröffentlichungsnummer: WO 2004/041225

(56) Entgegenhaltungen:
- EP-A- 0 920 855
- DE-A- 2 833 989
- US-A- 5 886 044
- US-A- 5 886 044

## Beschreibung

Die vorliegende Erfindung betrifft Mittel zur oxidativen Färbung von Keratinfasern, insbesondere menschlichen Haaren, welche 2-Amino-5-ethylphenol als Farbkomponente enthalten.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 1,4-Diaminobenzol und 4,5-Diamino-1-(2-hydroxyethyl)-pyrazol eingesetzt, während als Kupplersubstanzen beispielsweise Resorcin, 2-Methyl-resorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor toluol zu nennen sind.

An Oxidationsfarbstoffe, die zur Färbung menschlicher Haare verwendet werden, sind neben der Stabilität der Haarfärbungen über mindestens 4 bis 6 Wochen zahlreiche zusätzliche Anforderungen gestellt. So müssen die Farbstoffe in toxikologischer und dermatologischer Hinsicht unbedenklich sein und die erzielten Haarfärbungen sollen eine gute Lichtechtheit, Dauerwellechtheit, Reibeechtheit und Stabilität gegenüber Schampoonierung sowie eine ausreichende Beständigkeit gegenüber Schweißabsonderungen aufweisen. Außerdem ist es erforderlich, dass durch Kombination geeigneter Entwicklersubstanzen und Kupplersubstanzen eine breite Palette verschiedener Farbnuancen erzeugt werden kann.

Ein besonderes Problem stellt bei der Nuancierung von helleren Farbtönen die gleichmäßige Farbstoffaufnahme vom Haaransatz bis in die Haarspitzen sowie die Haltbarkeit der Färbungen gegenüber einer Dauerwellbehandlung dar. Die Verwendung von direktziehenden gelbfärbenden aromatischen Nitrofarbstoffen zusammen mit oxidativen Haarfarbvorstufen kann zwar die geschilderten Probleme zu einem Teil lösen, die Stabilität der Färbungen ist jedoch über einen Zeitraum von mehreren Wochen oft unbefriedigend.

Zu der Lösung des geschilderten Problems wird in der DE 28 33 989 A1 die Verwendung von 2-Amino-5-methyl-phenol als gelbfärbender oxidativer Farbstoff in oxidativen Haarfärbemitteln vorgeschlagen. Diese Verbindung ist zwar gut geignet als Nuancierfarbstoff zur Erzeugung von hellen Blondtönen und Goldtönen, sie kann jedoch die gestellten Anforderungen vor allem im Hinblick auf die Beständigkeit der Haarfärbungen gegenüber der Einwirkung von chemischen Substanzen wie zum Beispiel Dauerwellmitteln nicht voll erfüllen.

Die EP 0 920 855 A1 beschreibt Haarfärbemittel, welche als Farbkomponente eine Kombination aus einem 3-Alkyl-4-aminophenol und einem 2-substituierten 1-Naphthol enthalten.

Es wurde nunmehr gefunden, dass bei der Verwendung von 2-Amino-5-ethylphenol enthaltenden Oxidationsfärbemitteln die an derartige Mittel gestellten Anforderungen, insbesondere in Bezug auf die Waschbeständigkeit und die Beständigkeit gegenüber der Einwirkung von chemischen Substanzen wie zum Beispiel Dauerwellmitteln in besonders hohem Maße erfüllt werden.

Gegenstand der vorliegenden Erfindung ist daher ein Mittel zur oxidativen Färbung von Keratinfasern, wie zum Beispiel Wolle, Pelzen, Federn oder Haaren und insbesondere menschlichen Haaren, welches dadurch gekennzeichnet ist, dass es 2-Amino-5-ethylphenol oder dessen physiologisch verträgliche, wasserlösliche Salze und mindestens einen Zusatzstoff, welcher ausgewählt ist aus der Gruppe bestehend aus Ethanol, Propanol, Isopropanol, Glycerin, Glykolen, Netzmitteln oder Emulgatoren aus den Klassen der anionischen, kationischen, amphoteren oder nichtionogenen oberflächenaktiven Substanzen, Verdickern und Pflegestoffen, enthält.

Das 2-Amino-5-ethylphenol kann sowohl als freie Base als auch in Form seiner physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Das 2-Amino-5-ethylphenol ist in dem erfindungsgemäßen Färbemittel in einer Gesamtmenge von etwa 0,001 bis 5 Gewichtsprozent enthalten, wobei eine Menge von etwa 0,001 bis 2 Gewichtsprozent und insbesondere 0,01 bis 1 Gewichtsprozent bevorzugt ist.

Das 2-Amino-5-ethylphenol färbt keratinisches Material ohne den Zusatz weiterer Farbstoffe in gelben Farbtönen.

Zur Erzielung weiterer Farbnuancen können zusätzlich übliche oxidative Farbstoffe, beispielweise Entwicklersubstanzen oder Kupplersubstanzen, alleine oder im Gemisch miteinander, zugesetzt werden.
Als Kupplersubstanzen kommen hierbei insbesondere N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxy-propoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1 -(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylaminophenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion in Betracht.
Als Entwicklersubstanzen kommen vorzugsweise 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diaminobenzol, 1,4-Diamino-2-(thiophen-2-yl)benzol, 1,4-Diamino-2-(thiophen-3-yl)benzol, 4-(2,5-Diaminophenyl)-2-((diethylamino)methyl)thiophen, 2-Chlor-3-(2,5-diaminophenyl)thiophen, 1,4-Diamino-2-(pyridin-3-yl)benzol, 2,5-Diaminobiphenyl, 2,5-Diamino-4'-(1-methylethyl)-1,1'-biphenyl, 2,3',5-Triamino-1,1'-biphenyl, 1,4-Diamino-2-methoxymethyl-benzol, 1,4-Diamino-2-aminomethyl-benzol, 1,4-Diamino-2-((phenylamino)methyl)benzol, 1,4-Diamino-2-((ethyl-(2-hydroxyethyl)-amino)methyl)benzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylaminoanilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 4-(((4-Aminophenyl)-methyl)-amino)anilin, 4-[(4-Amino-phenylamino)-methyl]-phenol, 1,4-Diamino-N-(4-pyrrolidin-1-yl-benzyl)-benzol, 1,4-Diamino-N-furan-3-ylmethyl-benzol, 1,4-Diamino-N-thiophen-2-ylmethyl-benzol, 1,4-Diamino-N-furan-2-yl-methyl-benzol, 1,4-Diamino-N-thiophen-3-ylmethyl-benzol, 1,4-Diamino-N-benzyl-benzol, 1,4-Diamino-2-(1-hydroxyethyl)-benzol, 1,4-Diamino-2-(2-hydroxyethyl)-benzol, 1,4-Diamino-2-(1-methylethyl-2-benzol, 1,3-Bis[(4-aminophenyl)(2-hydroxyethyl)amino]-2-propanol, 1,4-Bis[(4-Aminophenyl)-amino]-butan, 1,8-Bis(2,5-diaminophenoxy)-3,6-dioxaoctan, 2,5-Diamino-4'-hydroxy-1,1 '-biphenyl, 2,5-Diamino-2'-trifluormethyl-1,1'-biphenyl, 2,4',5-Triamino-1,1'-biphenyl, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-1-pentyl-1H-pyrazol, 4,5-Diamino-1-(phenylmethyl)-1H-pyrazol, 4,5-Diamino-1-((4-methoxyphenyl)methyl-1H-pyrazol, 2-Amino-phenol, 2-Amino-6-methyl-phenol, 2-Amino-5-methylphenol, 1,2,4-Trihydroxy-benzol, 2,4-Diaminophenol, 1,4-Dihydroxybenzol und 2-(((4-Aminophenyl)amino)methyl)-1,4-diaminobenzol in Betracht.

Die vorgenannten Entwicklersubstanzen und Kupplersubstanzen können in dem erfindungsgemäßen Färbemittel jeweils einzeln oder im Gemisch miteinander eingesetzt werden, wobei die Gesamtmenge an Entwicklersubstanzen und Kupplersubstanzen der in dem erfindungsgemäßen Mittel etwa 0,01 bis 12 Gewichtsprozent, insbesondere etwa 0,2 bis 6 Gewichtsprozent, beträgt.

Weiterhin kann das erfindungsgemäße Färbemittel zusätzlich andere Farbkomponenten, beispielsweise 4-(2,5-Diamino-benzylamino)-anilin oder 3-(2,5-Diamino-benzylamino)-anilin, sowie ferner übliche natürliche, naturidentische oder synthetische direktziehende Farbstoffe aus der Gruppe der anionischen (sauren) und kationischen (basischen) Farbstoffe, der Triarylmethanfarbstoffe, der Nitrofarbstoffstoffe, der Dispersionsfarbstoffe und der Azofarbstoffe enthalten, zum Beispiel natürliche Farbstoffe wie Indigo oder Henna, Triphenylmethanfarbstoffe wie 4-[(4'-amino-phenyl)-(4'imino-2",5"-cyclohexadien-1"-yliden)-methyl]-2-methyl-aminobenzol-monohydrochlorid (C.l. 42 510) und 4-[(4'-amino-3'-methyl-phenyl)-(4"-imino-3"-methyl-2",5"cyclohexadien-1 "-yliden)-methyl]-2-methyl-aminobenzol monohydrochlorid (C.l. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)-azo]-5hydroxy-naphthalin-1-sulfonsäure-Natriumsalz (C.l. 14 805) und Dispersionsfarbstoffe wie beispielsweise 1,4-Diaminoanthrachinon und 1,4,5,8-Tetraaminoantrachinon.

Das Färbemittel kann die vorgenannten anderen Farbkomponenten in einer Gesamtmenge von etwa 0,1 bis 4 Gewichtsprozent enthalten.

Die vorgenannten Entwicklersubstanzen und/oder Kupplersubstanzen und/oder anderen Farbkomponenten ermöglichen in Kombination mit dem erfindungsgemäßen 2-Amino-5-ethylphenol eine Vielzahl verschiedener Farbnuancen. So ist es beispielsweise durch Verwendung einer Kombination des erfindungsgemäßen 2-Amino-5-ethylphenols mit 4-(2,5-Diamino-benzyl-amino)-anilin und/oder 2-(3-Aminophenyl)-aminomethyl-1,4-diamino-benzol bzw. deren Salzen möglich, blonde bis braune Haarfärbungen zu erzielen.

Selbstverständlich können die zusätzlichen Kupplersubstanzen sowie die Entwicklersubstanzen und die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung, eine Paste, eine Creme, ein Gel, eine Emulsion oder eine Aerosolzubereitung sein. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak oder organischen Aminen, zum Beispiel Monoethanolamin und Triethanolamin, oder aber Aminosäuren oder anorganischen Basen wie Natriumhydroxid und Kaliumhydroxid, erfolgt. Ebenfalls ist es möglich Kombinationen der vorgenannten Verbindungen, insbesondere eine Kombination von Ammoniak und Monoethanolamin, zu verwenden. Für eine pH-Einstellung im sauren Bereich kommen anorganische oder organische Säuren, zum Beispiel Phosphorsäure, Essigsäure, Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und falls erforderlich mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an 2-Amino-5-ethylphenol ermöglicht Haarfärbungen mit ausgezeichneter Farbechtheit, insbesondere was die Lichtechtheit, Waschechtheit und Reibeechtheit sowie Dauerwellechtheit anbetrifft. Hinsichtlich der färberischen Eigenschaften bietet das erfindungsgemäße Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität und einen guten Farbausgleich zwischen geschädigtem und ungeschädigtem Haar aus. Die sehr guten färberischen Eigenschaften des Haarfärbemittels gemäß der vorliegenden Anmeldung zeigen sich weiterhin darin, dass dieses Mittel eine Anfärbung von ergrauten, chemisch nicht vorgeschädigten Haaren problemlos und mit guter Deckkraft ermöglicht.

Das 2-Amino-5-ethylphenol kann nach an sich bekannten Methoden durch Nitrierung von 3-Ethylphenol oder 3-Ethylphenol-Derivaten und anschließende Reduktion der Nitrogruppe zur Aminogruppe hergestellt werden. Eine entsprechendes Herstellungsverfahren wird beispielsweise in der WO 96/25157 A1 (Beispiel 92 a/b) beschrieben.

Die nachfolgenden Beispiele sollen den Gegenstand der Erfindung näher erläutern, ohne ihn darauf zu beschränken.

### Beispiele

### Beispiele 1 bis 15: Haarfärbemittel

Es werden Haarfärbelösungen der folgenden Zusammensetzung hergestellt:

| | |
|---|---|
| X g | 2-Amino-5-ethylphenol |
| U g | Entwicklersubstanz **E8** bis **E15** gemäß Tabelle 1 |
| Y g | Kupplersubstanz **K11** bis **K35** gemäß Tabelle 3 |
| Z g | direktziehender Farbstoff **D1** bis **D3** gemäß Tabelle 2 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Haarfärbelösung werden unmittelbar vor der Anwendung mit 30 g einer 6prozentigen wässrigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

### Beispiele 16 bis 21: Haarfärbemittel

Es werden cremeförmige Farbträgermassen der folgenden Zusammensetzung hergestellt:

| | |
|---|---|
| X g | 2-Amino-5-ethylphenol |
| U g | Entwicklersubstanz **E8** bis **E15** gemäß Tabelle 1 |
| Y g | Kupplersubstanz **K11** bis **K35** gemäss Tabelle 3 |
| Zg | direktziehender Farbstoff **D1** bis **D3** gemäss Tabelle 2 |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak 22%ige wässrige Lösung |
| 0,3 g | Natriumsulfit, wasserfrei |
| ad 100 g | Wasser |

30 g der vorstehenden Haarfärbecreme werden unmittelbar vor der Anwendung mit 30 g einer 6prozentigen Wasserstoffperoxidlösung vermischt. Anschließend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind in Tabelle 6 zusammengefasst.

### Beispiele 22 bis 29: Haarfärbemittel

Es werden Haarfärbelösungen der folgenden Zusammensetzung hergestellt:

| | |
|---|---|
| X g | 2-Amino-5-ethylphenol |
| Zg | Farbkomponente **W1** bzw. **W2** gemäss Tabelle 4 |
| U g | Entwicklersubstanz **E8** bis **E15** gemäß Tabelle 1 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Haarfärbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40°C wird das Haar mit Wasser gespült, mit einem handelsüblichen Shampoo gewaschen und getrocknet. Die Färbeergebnisse sind der nachfolgenden Tabellen 7 zu entnehmen.

**Tabelle 1:**

| **Entwicklersubstanzen** | |
|---|---|
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Aminophenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 2:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 3:**

| **Kupplersubstanzen** | |
|---|---|
| **K11** | 1,3-Diaminobenzol |
| **K12** | 2-Amino-4.-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylhamstoff |
| **K19** | 1,3-Bis(2,4-diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol*HCl |
| **K35** | 3,4-Methylendioxy-phenol |

**Tabelle 4:**

| **Farbkomponenten** | |
|---|---|
| **W1** | 4-(2,5-Diamino-benzylamino)-anilin*HCl |
| **W2** | 2-(3-Amino-phenyl)aminomethyl-1,4-diamino-benzol*HCl |

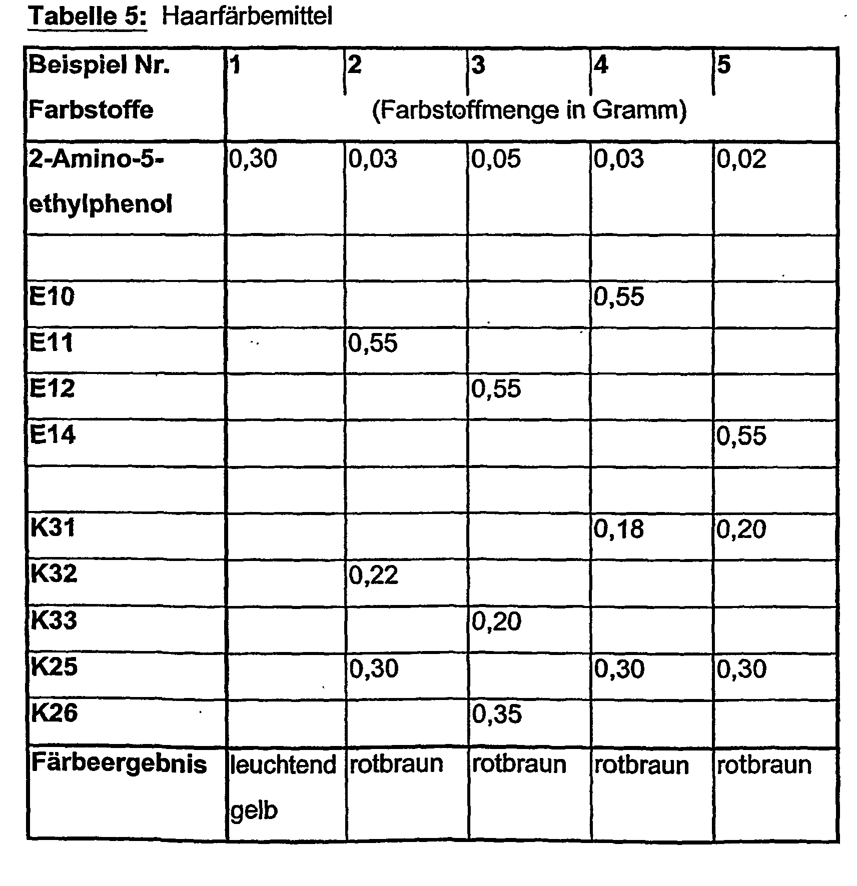

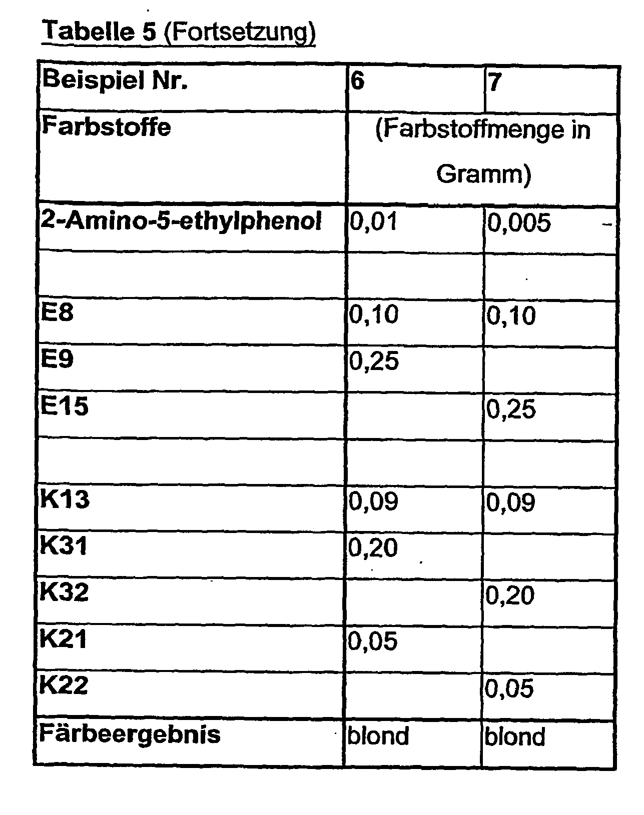

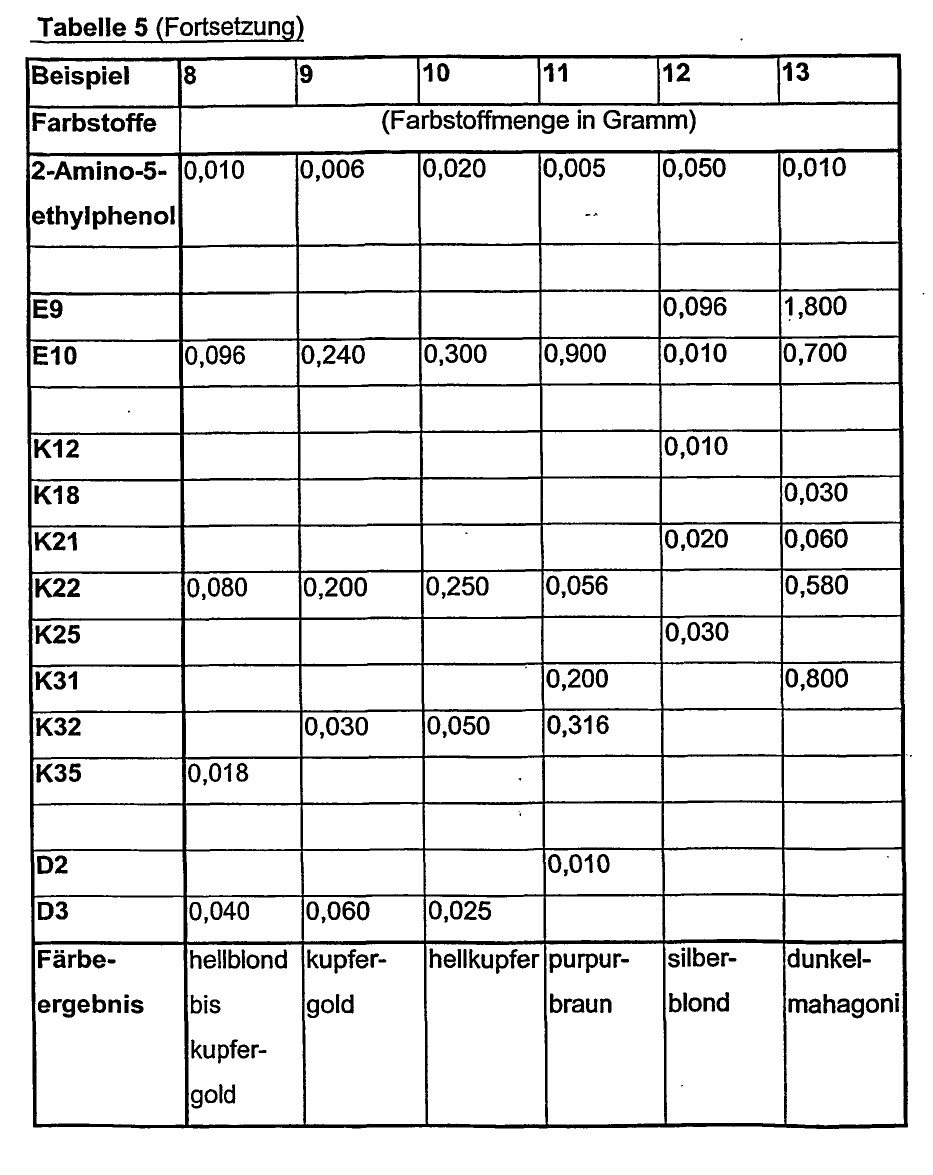

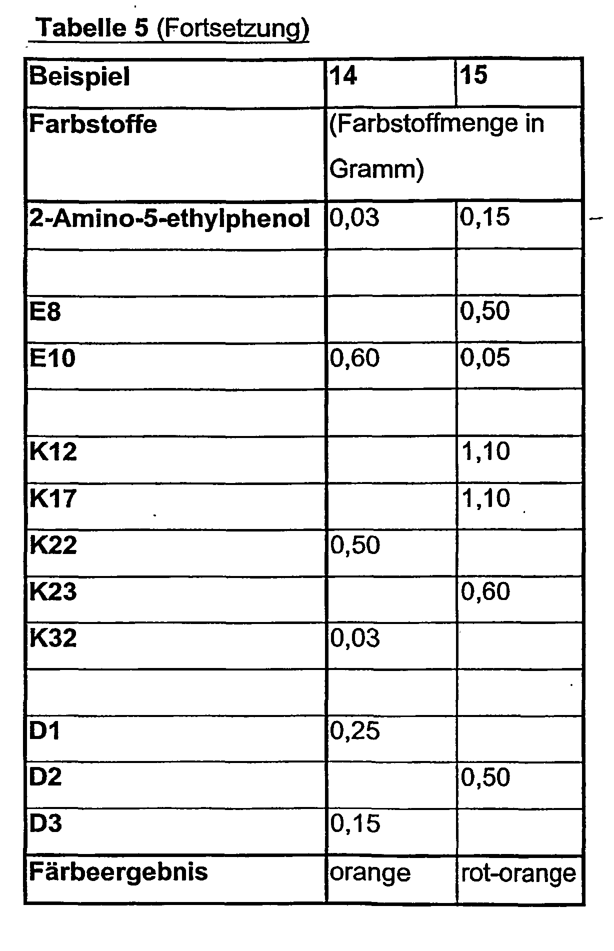

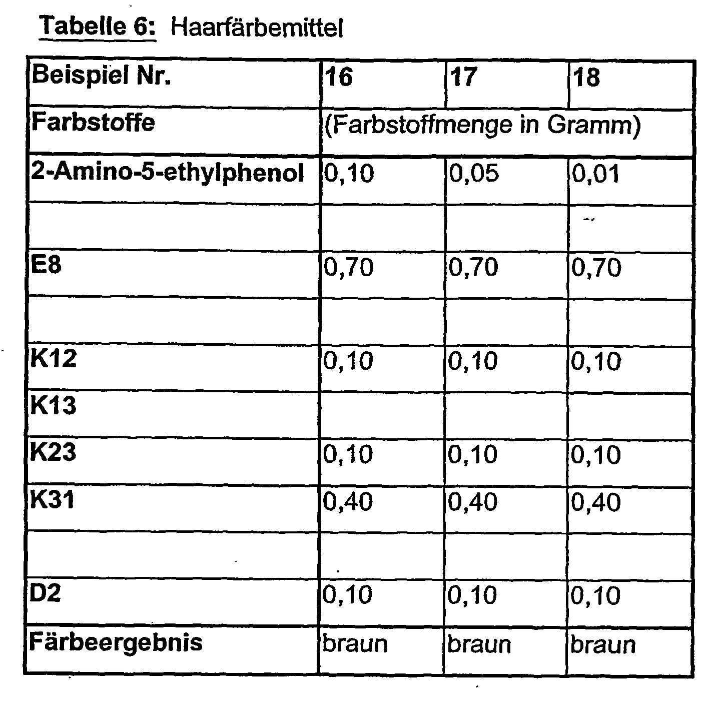

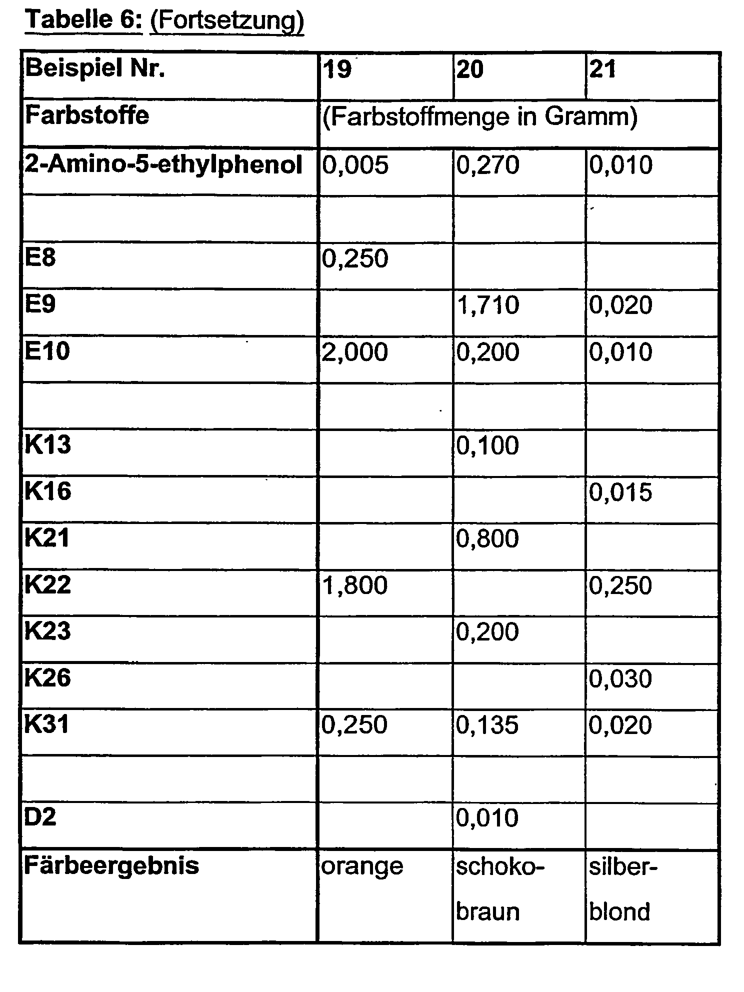

**Tabelle 7:**

| **Beispiel Nr.** | **22** | **23** | **24** | **25** | **26** | **27** | **28** | **29** |
|---|---|---|---|---|---|---|---|---|
| **Farbstoffe** | (Farbstoffmenge in Gramm) | | | | | | | |
| **2-Amino-5-ethylphenol** | 0,01 | 0,18 | 0,04 | 0,18 | 0,1.8 | 0,18 | 0,06 | 0,18 |
| | | | | | | | | |
| **E8** | | 0,12 | | 0,12 | | | | |
| **E9** | | | 0,12 | 0,15 | 0,15 | | | |
| **E15** | | | | | 0,13 | 0,13 | | |
| | | | | | | | | |
| **W1** | 0,90 | | | 0,38 | | 0,38 | 0,38 | 0,38 |
| **W2** | | 0,37 | 0,05 | | 0,58 | | | |
| **Färbeergebnis** | tiefblau | mittelbraun | mittelblond | schwarzbraun | braun | schwarzbraun | mittelbraun | braun |

Alle in der vorliegenden Anmeldung enthaltenen Prozentangaben stellen soweit nicht anders angegeben Gewichtsprozente dar.

## Patentansprüche

1. Mittel zur oxidativen Färbung von Keratinfasern, **dadurch gekennzeichnet, dass** es 2-Amino-5-ethylphenol oder dessen physiologisch verträgliche, wasserlösliche Salze, und mindestens einen Zusatzstoff, welcher ausgewählt ist aus der Gruppe bestehend aus Ethanol, Propanol, Isopropanol, Glycerin, Glykolen, Netzmitteln oder Emulgatoren aus den Klassen der anionischen, kationischen, amphoteren oder nichtionogenen oberflächenaktiven Substanzen, Verdickern und Pflegestoffen, enthält.

2. Mittel nach Anspruch 1, **dadurch gekennzeichnet, dass** das 2-Amino-5-ethylphenol in einer Menge von 0,001 bis 5 Gewichtsprozent enthalten ist.

3. Mittel nach Anspruch 1 oder 2 , **dadurch gekennzeichnet, dass** es zusätzlich mindestens einen Farbstoff aus der Gruppe bestehend aus Entwicklersubstanzen, Kupplersubstanzen, direktziehenden Farbstoffen und anderen Farbkomponenten enthält.

4. Mittel nach Anspruch 3, **dadurch gekennzeichnet, dass** der zusätzliche Farbstoff ausgewählt ist aus 4-(2,5-Diamino-benzyl-amino)-anilin und 2-(3-Aminophenyl)-aminomethyl-1,4-diamino-benzol sowie deren Salzen.

5. Mittel nach einem der Ansprüche 1 bis 4, **dadurch gekennzeichnet, dass** es einen pH-Wert von 6,5 bis 11,5 aufweist.

6. Verfahren zum oxidativen Färben von Haaren, **dadurch gekennzeichnet, dass** man vor der Anwendung ein Haarfärbemittel nach einem der Ansprüche 1 bis 4 mit einem Oxidationsmittel vermischt, sodann auf das Haar aufträgt, bei einer Temperatur von 15 bis 50 °C 10 bis 45 Minuten lang einwirken lässt, das Haar anschliessend mit Wasser ausspült, gegebenenfalls shampooniert und sodann trocknet.

## Claims

1. A colorant for oxidative coloring of keratin fibers, **characterized in that** it contains 2-amino-5-ethylphenol or the physiologically compatible, water-soluble salts thereof, and at least one additive selected from the group consisting of ethanol, propanol, isopropanol, glycerin, glycols, wetting agents or emulsifiers from the classes of the anionic, cationic, amphoteric or non-ionogenic surface-active substances, thickeners and conditioners.

2. The colorant according to claim 1, **characterized in that** the 2-amino-5-ethylphenol is present in an amount from 0.001 to 5 percent by weight.

3. The colorant according to claim 1 or 2, **characterized in that** it additionally contains at least one dye from the group consisting of developer substances, coupler substances, direct dyes and other dye components.

4. The colorant according to claim 3, **characterized in that** the additional dye is selected from 4-(2,5-diamino-benzylamino)-aniline and 2-(3-aminophenyl)-aminomethyl-1,4-diaminobenzene and the salts thereof.

5. The colorant according to one of claims 1 to 4, **characterized in that** it has a pH from 6.5 to 11.5.

6. A method for oxidative coloring of hair, **characterized in that**, before use, a hair colorant according to one of claims 1 to 4 is mixed with an oxidant, applied to the hair, allowed to act at a temperature from 15 to 50°C for 10 to 45 minutes, after which the hair is rinsed with water, optionally shampooed and then dried.

## Revendications

1. Agent de coloration oxydative de fibres de kératine, **caractérisé en ce qu'**il contient du 2-amino-5-éthylphénol ou des sels hydrosolubles physiologiquement de celui-ci, et au moins un additif, lequel est choisi parmi un groupe comprenant l'éthanol, le propanol, l'isopropanol, le glycérol, des mouillants ou des émulsifiants provenant des classes des substances, épaississants et agents de soins anioniques, cationiques, amphotères ou non-ioniques.

2. Agent selon la revendication 1, **caractérisé en ce que** le 2-amino-5-éthylphénol est contenu dans une quantité de 0,001 à 5 % en poids.

3. Agent selon la revendication 1 ou 2, **caractérisé en ce qu'**il contient en outre au moins un colorant parmi le groupe comprenant les agents développateurs, les agents coupleurs, les colorants montant directement sur les fibres et d'autres composants de couleur.

4. Agent selon la revendication 3, **caractérisé en ce que** le colorant supplémentaire est choisi parmi le 4-(2,5-diaminobenzylamino)aniline et le 2-(3-aminophényl)aminométhyl-1,4-diaminobenzène et leurs sels.

5. Agent selon l'une des revendications 1 à 4, **caractérisé en ce qu'**il présente une valeur pH de 6,5 à 11,5.

6. Procédé de coloration oxydative de cheveux, **caractérisé en ce qu'**avant l'application, on mélange un agent de coloration de cheveux selon l'une des revendications 1 à 4 avec un agent d'oxydation, qu'on applique ensuite sur les cheveux, qu'on laisse agir à une température de 15 à 50 °C pendant 10 à 45 minutes, qu'on rince ensuite les cheveux à l'eau, qu'on shampouine éventuellement, puis qu'on sèche.
